# EUROPEAN PATENT SPECIFICATION

(11) **EP 0 839 024 B1**
(45) Date of publication and mention of the grant of the patent: **21.04.2004**
(21) Application number: 96923112.5
(22) Date of filing: 04.07.1996
(51) Int. Cl.: A61K 9/10, A61K 47/36, A61K 9/00, A61K 31/35

(54) **MONENSIN FORMULATIONS**
FORMULIERUNGEN MIT MONENSIN
FORMULATIONS DE MONENSINE

(30) Priority: 14.07.1995 NZ 27257495; 06.09.1995 NZ 27294095
(43) Date of publication of application: 06.05.1998
(73) Proprietor: Elli Lilly & Co.(NZ)Limited, Manukau City, Auckland (NZ)
(72) Inventor: MOORE, Derek, George, Auckland (NZ); LOWE, Lionel, Barry, Dural, NSW 2158 (AU); PALMER, Kevin, Charles, Auckland (NZ); AGNEW, Kim, Ewing, Melville, Auckland (NZ)
(74) Representative: Kent, Lindsey Ruth
(86) International application number: PCT/NZ1996/000068
(87) International publication number: WO 1997/003650

(56) References cited:
- EP-A- 0 405 930
- EP-A- 0 470 667
- EP-A- 0 556 057
- EP-A- 0 620 001
- US-A- 4 746 349
- US-A- 5 112 604
- US-A- 5 300 439
- CYTOMETRY, Volume 10(6), 1989, "Use of Xantham Gum to Suspend Large Particles During Flow Cytometric Analysis and Sorting", FREYER, J.P. et al., pages 803-806.

## Description

### TECHNICAL FIELD

The present invention relates to an aqueous base suspension formulation of the ionophore antibiotic monensin capable of being administered to an animal.

Administration of ionophore antibiotics such as monensin to animals (preferably ruminants) is known to achieve in appropriate dosages advantages for a number of different purposes. These include the treatment or prevention of ketosis and/or bloat, the enhancement of milk production, enhancement of milk protein content in milk, enhancement of mineral uptake, enhancement of weight gain, and/or enhancement of feed conversion efficiency (in ruminants), desirable reproduction advantages and as a milk replacement. See US Patent 3,829,557.

### BACKGROUND ART

In this respect we refer to European Patent Specification 0,139,595 A2 of KOFFOLK (1949) LTD which relates to a liquid ionophore antibiotic composition for ruminants and poultry where the antibiotic is dissolved in a non-toxic water-soluble organic solvent and in use the resulting solution is admixed with a liquid feed, a liquid vitamin concentrate or drinking water. There are claims for stability on standing even for long periods.

The composition of EP 0,139,595 indicates that because monensin and its sodium salt is only slightly soluble in water that it is generally administered in a dry form in an animal feed and/or in dry liquid milk replacer compositions. The same is also indicated as being true for another ionophore antibiotic lasalocid which in US Patent 3,715,372 is reported to be completely insoluble in water.

The composition of EP 0,139,595 uses as an organic solvent for the ionophore antibiotic a solvent selected from the group comprising propylene glycol, glycerol ethanol and isopropanol and mixtures thereof.

Example 1 of EP 0, 139,595 indicates that 250 g of a mycelium containing 10% monensin was mixed at room temperature with 1250 cc propylene glycol for 2 hours by exposing the mixture to ultrasound. There is an indication that 50% of the monensin was found in solution in the propylene glycol.

Monensin is usually available commercially as the sodium salt of the acid. Monensin sodium is available in two forms, namely, a crystalline form or a mycelial form. The mycelial form has only about a 20% activity while the crystalline form has greater than 90% active monensin sodium.

Reference herein to "monensin" where the context allows encompasses all forms thereof including monensin, alkali metal salts of monensin and monensin esters. Likewise for the other ionophore antibiotics.

We have found monopropylene glycol to be a poor solvent of monensin and therefore while we refer to monopropylene glycol as being an antifreeze agent in which the ionophore antibiotic is sparingly soluble we do appreciate that solubility to the extent referred to in Example 1 of EP 0,139,595 may be achieved when induced with ultrasound or heating.

We have found however that it is possible to include in an aqueous suspension system monopropylene glycol as an antifreeze agent without any significant uptake of the stably suspended monensin from the aqueous system into the organic antifreeze agent. This is despite the fact that an organic solvent, such as methanol, allows monensin to crash out of solution if any water is added.

An organic antifreeze agent is deemed desirable in any such aqueous base suspension concentrate since frequently such concentrates are stored in sheds that are subject to extreme variations in temperature including temperatures below the freezing point of the aqueous system unless enhanced with a antifreeze agent.

Any freezing and thawing of the mixture is deemed undesirable as it may affect the uniformity of the monensin or other ionophore antibiotic distribution in the aqueous system. Thus the desirability of the inclusion of an antifreeze agent.

### DISCLOSURE OF INVENTION

The present invention therefore in at least one aspect is directed to an aqueous base suspension formulation that can stably support the ionophore antibiotic, monensin, with at least the majority of the monensin being within the aqueous system and not in any organic inclusion that has been added in as an antifreeze agent.

We have also found (irrespective of whether or not any antifreeze agent is present) that monensin can be stably suspended in aqueous suspension by Xanthan Gum that (i) holds the suspension stable when not subjected to shear but which shear thins, (ii) does not significantly age thicken, (iii) is capable of stably supporting a wide range of trace elements of biological significance (especially if in a chelated form) and (iv) is microbiologically stable.

The present invention therefore envisages an aqueous base suspension concentrate that stably supports the ionophore antibiotic, monensin, at least in part with Xanthan Gum.

We have also found that as a wetting agent for enabling the mixing of monensin was much easier using an alkyl polyglycoside over other wetting agents.

The present invention therefore envisages an aqueous base suspension formulation that stably supports the ionophore antibiotic, monensin, where at least some of the antibiotic was mixed with water in the presence of alkyl polyglycoside.

It is believed that an aqueous based suspension formulation in accordance with the present invention together with its diluted form and its use will provide the public with a useful choice.

In a first aspect the invention consists in an aqueous base suspension formulation of the ionophore antibiotic, monensin, capable of being orally administered to a ruminant animal (eg; by drenching), said formulation comprising
Monensin;
An alkyl polyglycoside;
Monopropylene glycol;
Xanthan gum; and
Water.
The formulation of the present invention may also further comprise one or more of
(i) an antifoam agent or system;
(ii) a preservative;
(iii) a de-bittering agent; or
(iv) a pH buffering system.
Preferably one or more (preferably all) of (i), (ii), (iii) and (iv) are present.

Preferably said monensin is present in an amount effective
(A) in beef and dairy cattle, in the treatment or prevention of Ketosis and/or bloat, in the enhancement of milk production, enhancement of milk protein content in milk, enhancement of mineral uptake, enhancement of weight gain, and/or enhancement of feed conversion efficiency in the treatment or prevention of coccidiosis, and/or
   in effecting reproduction advantages, and/or
(B) in calves for any of the purposes of (A) and/or
   as, at least in part a milk replacer or additive, and/or
   in the treatment or prevention of coccidiosis, and/or
(C) in a ruminant, for combination with and/or substantially simultaneous coadministration with other drench components (eg, trace elements, MgO, magnesium salts, zinc salts, pluronics, alcohol ethoxylates, and other metabolic additives) while exhibiting a sufficiently effective role selected from (A) and/or (B).

Preferably said Monensin is Sodium Monensin.

Preferably the amount of the monensin is from 3% w/v to 50% w/v (preferably 3 - 20% w/v and most preferably about 6% w/v).

Preferably the monensin is in a crystalline and/or mycelial form.

Preferably said monopropylene glycol is not introduced into the concentrate during its formulation until after substantial stability of the monensin has already been established in the water or at least some of the water.

Preferably the formulation of the present invention includes a Simethicon (and carrier therefor) antifoam agent.

Preferably the antifoaming agent system is Simethicone and Silicon Dioxide (premixed together).

Preferably the formulation of the present invention includes a pH buffering system.

Preferably the buffering system is a phosphate buffering system.

Preferably the buffering system is Disodium Phosphate Anhydrous and Monopotassium phosphate Dihydrate.

Preferably the formulation of the present invention includes a preservative (preferably with wetting properties eg; Dialkyl dimethyl ammonium bromide).

Preferably the alkyl polyglycoside is added to water prior to addition of the monensin.
Preferably the formulation comprises:

| | | |
|---|---|---|
| Sodium Monensin (QA33 1Z) | 3% - 50% w/v | Ionophore antibiotic |
| Monopropylene glycol | 1% - 20% w/v | Antifreeze |
| Disodium Phosphate Anhydrous | 0% - 1% w/v | Buffer |
| MonoPotassium phosphate Dihydrate | 0% - 0.5% w/v | Buffer |
| Dialkyl dimethyl ammonium bromide | 0% - 0.1 % w/v | Preservative |
| Sorbitol | 0% - 10% w/v | Debittering agent |
| Xanthan Gum | 0.05% - 5% w/v | Suspension agent |
| Alkyl Polyglycoside | 0.05% - 2% w/v | Surfactant/Wetting agent |
| Simethicone | 0.05% - 2% w/v | Anti-foam |
| Silicon Dioxide | 0.01 % - 1 % w/v | Carrier for Simethicone |
| Water to 100% | | |

and most preferably is

| | | |
|---|---|---|
| Sodium Monensin | about 6% w/v | Ionophore antibiotic |
| Monopropylene glycol | about 10% w/v | Antifieeze |
| Disodium Phosphate Anhydrous | about 0.355% w/v | Buffer |
| MonoPotassium phosphate Dihydrate | about 0.04% w/v | Buffer |
| Dialkyl dimethyl ammonium bromide | about .0064% w/v | Preservative |
| Sorbitol | about 3.5% w/v | Debittering agent |
| Xanthan Gum | about 0.4% w/v | Suspension agent |
| Alkyl Polyglycoside | about 0.5% w/v | Surfactant/Wetting agent |
| Simethicone | about 0.333% w/v | Anti-foam |
| Silicon Dioxide | about 0.167% w/v | Carrier for Simethicone |
| Water to 100% | | |

Depending on Sodium Monensin batch purity actual amounts required to provide an effective 6%w/v have been 6.24%w/v and 6.33% w/v.

An alternative antifoam agent to the simethicone/silicon dioxide system is a dimethyl polysiloxane/polyoxyalkylene system [silicone glycols]. Where such an alterative is used quantities utilized are substantially as previously stated.

In still a further aspect the invention consists in a method of preparing an aqueous base suspension formulation of the ionophore antibiotic, monensin, capable of being orally administered to a ruminant animal (eg; by drenching), said formulation comprising:
(I) monensin in (II) an aqueous system containing
   (i) an alkyl polyglycoside;
   (ii) monopropylene glycol;
   (iii) Xanthan gum;
   (iv) optionally an antifoam agent or system;
   (v) optionally a preservative;
   (vi) optionally a de-bittering agent;
   (vii) optionally a pH buffering system; and
   (viii) water,
   which comprises
   mixing the monensin to an aqueous system already containing at least some monopropylene glycol, the optional preservative (if present and having a wetting effect), and the antifoaming agent or system, and
   subsequently mixing in the balance of components (optionally with some measure of premixing).

Preferably the method of preparation is substantially as herein after described.

The formulation of the present invention is of use in a method of treating a ruminant animal for any one or more of the conditions previously set forth or for any of the purposes previously set forth and/or as hereinafter described which comprises administering to the animal orally, or allowing self administration orally, of an aqueous base suspension formulation in accordance with the present invention.

Preferably said administration is by means of drenching, eg; preferably using drenching apparatus.

Preferably said administration is of sodium monensin at a daily rate per ruminant animal of from 0.4 milligrams per Kg body weight of animal to 2 milligrams per Kg body weight of animal.

Reference herein to formulations does not rule out the inclusion therein of compatible other inclusions such as essential minerals etc that can usefully be administered simultaneously with the monensin. See for example Australian Patent Specification No. 270639 filed 6 March 1995.

### BRIEF DESCRIPTION OF DRAWINGS

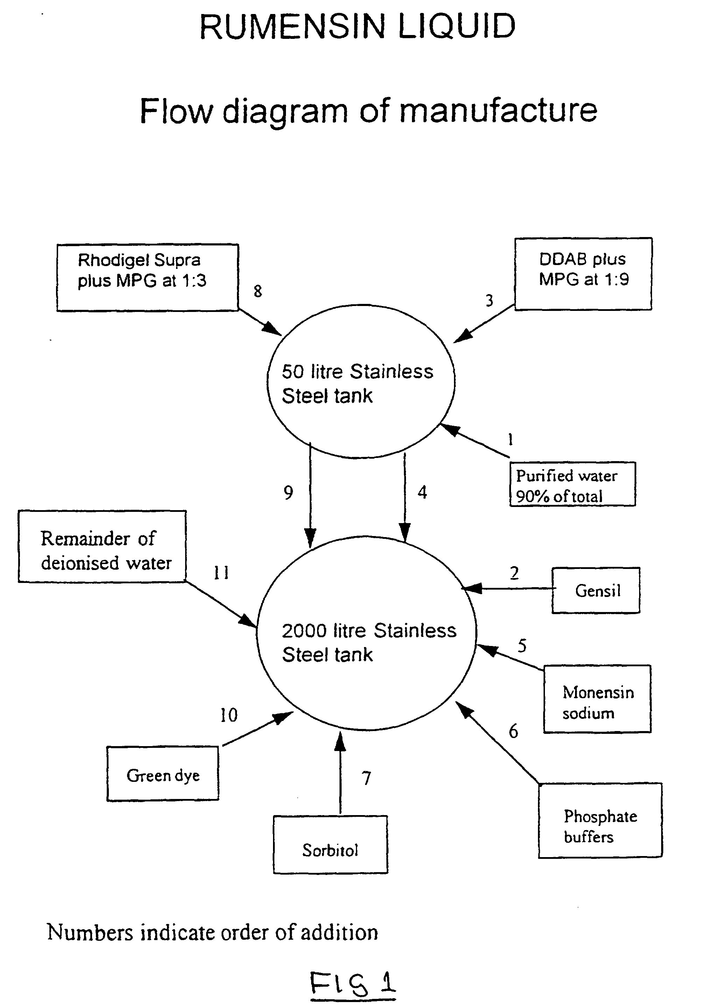

Preferred forms of the present invention will now be described with reference (where appropriate) to the accompanying drawings in which Figure 1 is a flow diagram showing by reference to the numbers a preferred sequence of addition of specified componentary into one or other of two mixing containers, the numerical sequence being the order of addition.

Preferred forms of the present invention will be described with reference to monensin. With particular reference to crystalline sodium monensin about a 6%w/v inclusion is referred to. This equates to about 6.33%w/v commercially available sodium monensin) because of impurities.

It has primarily been the intention however to utilise sodium monensin and solubilising of the monensin was rejected, as was the formation of a wettable powder formulation in favour of a very stable aqueous base suspension.

In evolving the formulations of the present invention a great number of different wetting agents were tried to ensure an adequate wetting of the monensin when being mixed with water. In this respect lecithins, sodium lauryl sulphate, sodium dodecyl sulphate and marlon was tried. Lecithins did not work well, sodium lauryl sulphate and marlon did work but were found not to be as effective as wetting agents as alkyl polyglycoside.

Attempts to solubilise monensin in an organic solvent such as methylated spirits prior to introduction to water was rejected owing to the effect that the alcohols had on at least some suspension agents, ie. guar gum when added was precipitated out by the presence of the organic solvent. Yet when monensin in methylated spirits was added to the water alone the monensin itself precipitated out.

Attempts were made to utilise Mg(OH)₂ + Al(OH)₃ but it was found that the use thereof in conjunction with monensin led to inadequate suspension of the monensin in the aqueous phase.

Notwithstanding the reference above to attempts to use lecithins as wetting agents, lecithin type compounds such as Emulfuid E, Blendmax 322 and Yelkinol P were used as wetting agents for the monensin. Some were enzyme modified compounds utilized with a view to modifying the Hydrophilic/Lipophilic balance (ie. HLB) of the compounds. Notwithstanding such attempts as indicated earlier the lecithins did not allow for an optimum wetting of monensin when compared with, by way of example alkyl polyglycoside. Moreover the lecithins tended to be oily and powdered lecithins were expensive.

Cellulose type compounds such as methocel and cellogen were also utilised as possible thickeners in an attempt to hold monensin in aqueous suspension. Some separation was found with such cellulose type compounds with the monensin settling out. Such cellulose type compounds were prone also to age thickening as also was the case with guar gum.

Other suspension agents and wetting agents were utilized, for example, liposorb. Nothing however was located that was as effective as a wetting agent as alkyl polyglycoside nor as effective as a suspension agent as xantham gum.

The present invention relates to an aqueous base suspension formulation capable of being administered by a drench gun, such as for example, the DRENCHMATIC™ of Instrument Supplies of Comer Bryant Road and Vickery Street, Te Rapa, Private Bag 3126, Hamilton, New Zealand or the PNEUMEDIC™ Drench Gun System of Engineering Dynamics Ltd of PO Box 30-812, Lower Hutt, New Zealand.

In each of these systems it is possible to administer the aqueous base suspension formulation of the present invention. The present invention relates to a stable suspension having a shelf life sufficient to allow dilution and then drench administration for an extended period with a minimum of mixing difficulties at the time of dilution and little in the way of metering difficulties to ensure an effective dosage of the ionophore antibiotic is being given.

The preferred suspension in accordance with the present invention is as follows

| | | |
|---|---|---|
| Sodium Monensin | 6.33% (about 6%) w/v | Ionophore antibiotic |
| Monopropylene glycol | 10% w/v | Antifreeze |
| Disodium Phosphate Anhydrous | 0.355% w/v | Buffer |
| MonoPotassium phosphate Dihydrate | 0.04% w/v | Buffer |
| Dialkyl dimethyl ammonium bromide | .0064% w/v | Preservative |
| Sorbitol | 3.5% w/v | Debittering agent |
| Xanthan Gum | 0.4% w/v | Suspension agent |
| Alkyl Polyglycoside | 0.5% w/v | Surfactant/Wetting agent |
| Simethicone | 0.333% w/v | Anti-foam |
| Silicon Dioxide | 0.167% w/v | Carrier for Simethicone |
| Water to 100% | | |

The preferred form of the present invention however administers monensin and specifically sodium monensin in its crystalline form.

Crystalline monensin can be prepared in a number of different ways. Such a product is available from Eli Lilly & Co, Indianapolis, USA or can be prepared by way of the procedures set out, by way of example, in US Patent 4,213,966 (E.R. Squibb & Sons Inc).

For stability of the ionophore antibiotic an alkaline pH and preferably one about 8 - 8.2 is desired. The phosphate solvent system preferably buffers for this pH range.

### The components:

### 1. Antifreeze Agent

Monopropylene glycol (MPG). It was found that it functioned adequately as an antifreeze.

Was useful to dissolve as a "premix" with DDAB (dialkyl dimethyl ammonium bromide - a preservative with some wetting agent characteristics) and xanthan gum (the preferred aqueous suspension agent for monensin).

Monensin was only very sparingly soluble in MPG without ultrasonic mixing and/or heating.

Since MPG works well and is safe and well known and used in the animal health industry, it is the antifreeze of choice.

### 2 The Suspension Agent

The suspension of choice is xantham gum, (a hydrocolloid) which has the advantage of locking the suspension when not mobilised but soon as movement is required it shear thins. Unlike many other gums, it also does not age thicken.

Xanthan gum was found to be far more effective than guar gum and other such gums which tended to produce a solid lump in the bottom of the container after a period of time.

Xanthan gum was also very stable with respect to ions such as MGO, ZNO. Moreover xanthan gum is microbiologically stable.

### 3. The Ionophore Antibiotic

Monensin as sodium monensin was the preferred ionophore antibiotic.

### 4. The Buffering System

The buffers are of any effective compatible buffering system of a kind set forth.

### 5. The Preservative

The preservative DDAB was preferably added into the formulation early so as to make best use of its wetting effect in addition to its intended preservative effect. Its presence provides broad spectrum activity from the preservative point of view while at the same time providing some measure of antibloat activity due to its wetting effect.

### 6. The Wetting Agent

The preferred wetting agent was clearly alkyl polyglycoside. Its molecular structure was most compatible with monensin and enabled monensin to enter the aqueous phase easier than other agents tried. It again is a component to be added early into the preparative process.

### 7. The Antifoaming Agent

The preferred anti foaming agent is preferably gensil, an emulsion of simethicone and silicon dioxide.

In the examples hereinafter set out of various formulations produced, all percentages are on a w/v basis and in each instance the formulations were of a kind intended to provide an about 3%w/v or about 6%w/v sodium monensin content. **Ingredients:** As in each Example.
**Equipment:** Water deioniser
Sanitary pump and connecting hose
2250 litre SS mixing tank (high speed agitator)
50 litre SS vessel

### Procedure (where ingredients of the Example allows):

1. Preweigh all raw materials, except water, as specified on worksheet.
2. Add approximately 90% of deionised water to stainless mixing tank and turn on high speed stirrer.
3. Add gensil (antifoam), rinse container with deionised water.
4. Add Agrimul 2067. Rinse container with a small amount of deionised water.
5. Preblend, in the 50 litre SS vessel, the DDAB at approximately 1:9 with monopropylene glycol and add to the blender.
6. Slowly add sodium monensin allowing powder to 'wet' before adding more. Once wetted, rinse down sides and stirrer to remove any powder.
   NB. If sodium monensin is added too quickly lumping may occur.
7. Add disodium phosphate and monopotassium phosphate and stir until dissolved.
8. Add sorbitol and stir until well mixed in (5 minutes).
9. Add 50% of the monopropylene glycol.
10. Preblend, in the 50 litre SS vessel, Rhodigel Supra at approximately 1 :3 monopropylene glycol and add to blender with remainder of MPG.
11. Add green dye.
12. Rinse sides and stirrer with deionised water and add balance of water up to mark.
13. Continue stirring until product has thickened and free from lumps (approximately 15-30 minutes). Turn off stirrer. NB. Product will have thickened adequately such that when stirrer is turned off, product will "spring back".

### Mix Sequence:

The mix sequence is such that preferably
(a) DDAB when present is added early but is mixed with monopropylene glycol for adding to the large tank.
(b) The antifoam suspension (preferably gensil) is added early in the sequence to minimise excessive foam being generated by the vigorous mixing required to wet monensin.

The addition of monensin late in the mix sequence is to be avoided, as the concentration of monensin achieved with such late addition is far more variable especially when dealing with concentrations greater than 5%w/v. With concentrations of sodium monensin of about 10%w/v it became increasingly difficult to incorporate monensin into the formulation.
The preferred sequence for a composition as previously stated or as set out in, for example, Example 3 is as described in Figure 1.

### Example 1

| | |
|---|---|
| Sodium Monensin | 6.24% |
| Sodium Lauryl Sulphate | 0.10% |
| Monopropylene Glycol FI | 8.00% |
| Sorbitol 70% USP FI | 1.00% |
| Xanthan Gum 2% Solution | 0.2% |
| Alkyl Polyglycosides | 0.20% |
| Antifoam Powder | 0.20% |
| Water | 79.876% |

### Example 2

| | |
|---|---|
| Sodium Monensin | 6.5% |
| Monopropylene Glycol FI | 7.5% |
| Disodium Phosphate Anhyd FI | 0.355% |
| Dipotassium Phosphate DI FI | 0.04% |
| Bromosept (DDAB 80) | 0.0064% |
| Sorbitol 70% USP FI | 5% |
| Xanthan Gum 2% Solution | 0.4% |
| Alkyl Polyglycosldes | 0.5% |
| Antifoam Powder | 0.5% |
| Water | 79.5586% |

### Example 3

| | |
|---|---|
| Sodium Monensin | 6.32% |
| Monopropylene Glycol FI | 10.0% |
| Disodium Phosphate Anhyd FI | 0.255% |
| Monopotssum Phosphate DI FI | 0.040% |
| Bromosept (DDAB 80) | 0.0064% |
| Sorbitol 70% USP FI | 5.0% |
| Xantham (Rhodigel Supra) FI | 0.40% |
| Agrimul APG 600 | 0.50% |
| Antifoam Powder | 0.50% |
| Water | 76.8786% |

### REPORT ON PERFORMANCE OF 6% (NOMINAL) MONENSIN SUSPENSION IN DRENCHING SYSTEMS

In all *in vivo* tests 1 litre of 6% monensin composition (as in Example 3) was added to 19 litres of water in the powder drenching system reservoir. This equates to a drenching rate of 100mls of drench providing 300mg monensin per day. The monensin suspension dispersed easily with a low level of foaming even on full agitation with the submersible pump on full by-pass.
1. To the 20 litres of monensin suspension was added the equivalent amount of nonylphenol ethoxylate (Blocare 4511) for dosing 200 cows at the rate of 5mls/cow. The foam level increased dramatically but this was due to the Blocate. No chemical reactions were observed.
2. To another 20 litre batch ofmonensin suspension was added 4kg of magnesium oxide (calmag). There was a slight increase in foam level but not more than is normal for calmag on its own. No chemical reactions were observed.
3. To another 20 litre batch of monensin suspension was added 20kg of magnesium chloride flake. There was no significant increase in foam. There was some sediment in the bottom of the reservoir on emptying. This was a mixture of undissolved MgCl₂ residual CALMAG™ (ie. MgO) and rust). There did not appear to be any chemical reactions.

### General Comments

Larger particles only of sodium monensin were seen to settle out 15 to 20 minutes after agitation cease. These particles re-suspended easily upon recommencement of liquid flow through the tubing (ie. agitation).

The settling out of the monensin was not considered to be a major problem because of
- the ease of re-suspension of the monensin, once re-agitated
- the degree of agitation provided by a power drench system
- by emptying the drench line prior to the start of a row of animals to be drenched the probability of an animal receiving more than two times the required dosage of monensin within the 100ml volume is very low.

### Evaluation of the effectiveness of 100 ml/day of Composition of 6%w/v sodium monensin to control bloat in milking cows on pasture

Two trials were carried out in September/October 1995 to evaluate the effectiveness of 100 ml/day of the Example 3 composition as a drench to control bloat in milking cows on pasture, firstly by drenching 50ml twice a day, ie. before each milking (Trial 1A), and secondly, by drenching 100ml before each morning milling (Trial 1B). There was 300mg active ingredient administered per day. In each Trial half of the 30 cows were allocated by restricted random allocation to the untreated Control group, and half to the Liquid Product ('treated') group. Animals were scored for bloat twice daily on white clover/ryegrass pastures or on red clover pastures (using a scale of 0, 1, 1.5, 2, 2.5, 3, representing scores of increasing severity of bloat, by assessing pressure on the cows' left and right sides).

In Trial 1A, serious bloat was recorded on 9 mornings out of 23 am and pm recording sessions. Twelve of 15 cows from the Control group were *at risk* of death from bloat (if left untreated) on at least one day, compared with 3 of 15 in the Treated group, a statistically significant difference (P<0.01). Mean scores on 8 bloating days were respectively 0.58 and 0.12 which was a significant difference (P<0.01). On the most serious four bloat days, corresponding scores were 0.94 and 0.20 (also a significant difference at P<0.01).

In Trial 1 B, 3 serious bloat sessions out of 9 am and pm recording sessions led to 8/15 Control animals being *at risk,* compared with 1/14 Treated animals (a statistically significant difference, P<0.01). Mean scores on these 3 bloating days were 0.65 and 0.10 respectively, representing a statistically significant difference between groups (P<0.01). The composition of Example 3 was successful in reducing the mean bloat score and the number of animals *at risk*.

### Trial 1A 25/9 to 6/10/95 Treatment = 50ml liquid Composition of 6%w/v sodium monensin twice a day

**Table 1.**

| Mean bloat score on days when bloat was recorded (treated and control) | | |
|---|---|---|
| **Day** | **Mean Score** | **Number of cows at risk (ie. A score ≥ 1.5)** |
| 1 | 0.27 | 4 |
| 3 | 0.08 | 1 |
| 4 | 0.08 | 1 |
| 5 | 0.09 | 1 |
| 8 | 0.18 | 1 |
| 9 | 0.70 | 9 |
| 10 | 0.85 | 11 |
| 11 | 0.37 | 5 |
| 12 | 0.47 | 5 |
| **Mean/Total** | **0.34** | **15*** |

| | | |
|---|---|---|
| * Excluding Day 1, where equilibration in the rumen may not have been reached. | | |

**Table 2.**

| Treatment effects on bloat-score means from all nine days when bloat was recorded, and from subsets of days | | | | | |
|---|---|---|---|---|---|
| **Number of score days** | **Non-treated** | **Treated** | **s.e.d.** | **Signif.** ^{**b**} | **Phenotypic s.d.** ^{**c**} |
| 9 ^{a} | 0.55 | 0.13 | 0.13 | ** | 0.60 |
| 8 (excluding first) | 0.568 | 0.12 | 0.13 | ** | 0.59 |
| Best 4 | 0.94 | 0.20 | 0.20 | ** | 0.73 |
| The Other 4 | 0.30 | 0.07 | 0.10 | * | 0.49 |

| | | | | | |
|---|---|---|---|---|---|
| ^{a} Between-cow repeatability = 0.28±0.07 (9 days); 0.45±0 10 (best 4 days). | | | | | |
| ^{b} *= P<0.05; ** = P<0 01 | | | | | |
| ^{c} From the cow and residual variance components combined. | | | | | |

**Table 3.**

| Numbers of bloated animals by treament (15 per group), using various criteria of bloat | | | | |
|---|---|---|---|---|
| **Criterion** | **Non-treated** | **Treated** | **Chi-square** | **Signif.**^{**b**} |
| Cows with a mean of 0 on Days 9-12 | 2 | 11 | 11.00 | *** |
| Cows with a mean ≥ on Days 9-12 | 7 | 1 | 6.14 | * |
| Cows at risk (score ≥ 1.5) on at least one day^{a} | 12 | 3 | 10.80 | ** |

| | | | | |
|---|---|---|---|---|
| ^{a} Excluding Day 1, where equilibration in the rumen may not have reached. | | | | |
| ^{b} * = P<0.05; II < P<0.01; ***= P<0.001. | | | | |

### Trial IB 24/10 TO 28/10/95 Treatment = 100ml liquid composition of 6%w/v sodium monensin per day in one dose at 0615

**Table 4.**

| Mean bloat score on days when bloat was recorded | | |
|---|---|---|
| **Day** | **Mean score** | **Number of cows at risk (ie. a score ≥ 1.5)** |
| 3 | 0.29 | 3 |
| 4 | 0.56 | 7 |
| 5 | 0.27 | 3 |
| **Mean/Total** | **0.37** | **9** |

**Table 5**

| Treatment effects on bloat score means from Days 3 to 5. | | | | | |
|---|---|---|---|---|---|
| **Trait** | **Non-treated** | **Treated** | **s.e.d.** | **Signif.** ^{**a**} | **Phenotypic**^{**b**} **s.d.** |
| Bloat score | 0.65 | 0.10 | 0.15 | *** | 0.60 |
| Number at risk (score ≥ 1.5) | 8 | 2 | 7.20^{c} | ** | |

| | | | | | |
|---|---|---|---|---|---|
| ^{a} **= P<0.01; *** = P<0.001. | | | | | |
| ^{b} Between-cow repeatability = 0.1±0.12 (3 days); the phenotypic standard deviation is from the cow and residual components combined. | | | | | |
| ^{c} Chi-square, testing 8/15 against 1/14. | | | | | |

## Claims

1. An aqueous base suspension formulation adapted for oral administration to a ruminant animal comprising.
monensin;
an alkyl polyglycoside;
monopropylene glycol;
Xanthan gum; and
water.

2. A formulation of Claim 1 further comprising one or more of
an antifoam agent or system;
a preservative;
a debittering agent; or
a pH buffering system.

3. A formulation of Claim 1 wherein said monensin is sodium monensin.

4. A formulation of Claim 1 wherein the amount of monensin is from 3% w/v to 50% w/v.

5. A formulation of Claim 4 wherein the amount of monensin is from 3% w/v to 20% w/v.

6. A formulation of Claim 5 wherein the amount of monensin is about 6% w/v.

7. A formulation of Claim 1 comprising
| | |
|---|---|
| Sodium Monensin | 3% - 50% w/v, |
| Monopropylene glycol | 1% - 20% w/v, |
| Xanthan Gum | 0.05% - 5% w/v, |
| Alkyl Polyglycoside | 0.05% - 2% w/v, |
| Simethicone | 0.05% - 2% w/v, |
| Silicon Dioxide | 0.01% - 1% w/v, and |
| Water to 100%. | |

8. A formulation of Claim 7 comprising
| | |
|---|---|
| Sodium Monensin | about 6% w/v, |
| Monopropylene glycol | about 10% w/v, |
| Disodium Phosphate Anhydrous | about 0.355% w/v, |
| Monopotassium Phosphate Dihydrate | about 0.04% w/v, |
| Dialkyl dimethyl ammonium bromide | about .0064% w/v, |
| Sorbitol | about 3.5% w/v, |
| Xanthan Gum | about 0.4% w/v, |
| Alkyl Polyglycoside | about 0.5% w/v, |
| Simethicone | about 0.333% w/v, |
| Silicon Dioxide | about 0.167% w/v, and |
| Water to 100%. | |

9. Use of a formulation according to any one of claims 1 to 8 in the manufacture of a medicament adapted for oral administration to a ruminant animal, which medicament is for
(A) in beef and dairy cattle,
the treatment or prevention of Ketosis and/or bloat,
the enhancement of milk production, enhancement of milk protein content in milk, enhancement of mineral uptake, enhancement of weight gain, and/or enhancement of feed conversion efficiency, the treatment or prevention of coccidiosis, and/or
effecting reproduction advantages, and/or
(B) in calves
any of the purposes of (A), and/or
a milk replacer or additive.

10. A method of forming a formulation according to any one of claims 1 to 8 which comprises mixing monensin to an aqueous system already containing at least some monopropylene glycol, the optional preservative (if present and having a wetting effect), and the antifoaming agent or system, and
subsequently mixing in the balance of components (optionally with some measure of premixing).

## Patentansprüche

1. Wässrige Basissuspensionsformulierung, die zur oralen Verabreichung an einen Wiederkäuer angepasst ist und umfasst
Monensin,
ein Alkylpolyglycosid,
Monopropylenglycol,
Xanthangummi, und
Wasser.

2. Formulierung nach Anspruch 1, die ferner eines oder mehrere umfasst aus
einem Antischaummittel oder Antischaumsystem,
einem Konservierungsmittel,
einem Bitterkeitsabschwächungsmittel, oder
einem pH Puffersystem.

3. Formulierung nach Anspruch 1, worin das Monensin Natriummonensin ist.

4. Formulierung nach Anspruch 1, worin die Menge an Monensin 3 % G/V bis 50 % G/V beträgt.

5. Formulierung nach Anspruch 4, worin die Menge an Monensin 3 % G/V bis 20 % G/V beträgt.

6. Formulierung nach Anspruch 5, worin die Menge an Monensin etwa 6 % G/V beträgt.

7. Formulierung nach Anspruch 1, die umfasst
| | |
|---|---|
| Natriummonensin | 3 - 50 % G/V, |
| Monopropylenglycol | 1 - 20 % G/V, |
| Xanthangummi | 0,05 - 5 % G/V, |
| Alkylpolyglycosid | 0,05 - 2 % G/V, |
| Simethicone | 0,05 - 2 % G/V, |
| Siliziumdioxid | 0,01 - 1 % G/V, und |
| Wasser | auf 100 %. |

8. Formulierung nach Anspruch 7, die umfasst
| | |
|---|---|
| Natriummonensin | etwa 6 % G/V, |
| Monopropylenglycol | etwa 10 % G/V, |
| Dinatriumphosphat, wasserfrei | etwa 0,355 % G/V, |
| Monokaliumphosphatdihydrat | etwa 0,04 % G/V, |
| Dialkyldimethylammoniumbromid | etwa 0,0064 % G/V, |
| Sorbit | etwa 3,5 % G/V, |
| Xanthangummi | etwa 0,4 % G/V, |
| Alkylpolyglycosid | etwa 0,5 % G/V, |
| Simethicone | etwa 0,333 % G/V, |
| Siliziumdioxid | etwa 0,167 % G/V, und |
| Wasser | auf 100 %. |

9. Verwendung einer Formulierung nach einem der Ansprüche 1 bis 8 zur Herstellung eines Arzneimittels, das zur oralen Verabreichung an ein wiederkäuendes Tier angepasst ist, wobei das Arzneimittel vorgesehen ist für
(A) Rinder und Milchkühe,
die Behandlung oder Prävention von Ketose und/oder Geblähtheit,
die Steigerung der Milchproduktion, Steigerung des Milchproteingehalts in Milch, Steigerung der Mineralaufnahme, Steigerung der Gewichtszunahme und/oder Steigerung der Futterkonversionseffizienz, Behandlung oder Prävention von Coccidiose und/oder
(B) die Bewirkung von Reproduktionsvorteilen und/oder
bei Kälbern für jeden der Zwecke von (A) und/oder
einen Milchaustauscher oder ein Additiv.

10. Verfahren zur Herstellung einer Formulierung nach einem der Ansprüche 1 bis 8, das das Mischen von Monensin in ein wässriges System, das zumindest bereits Monopropylenglycol, das optionale Konservierungsmittel (falls es vorkommt und einen benetzenden Effekt hat) und das Antischaummittel oder Antischaumsystem enthält und das anschließende Mischen zusammen mit den Komponenten (wahlweise mit Vormischmaßnahmen).

## Revendications

1. Formulation de suspension à base aqueuse conçue pour une administration orale à un animal ruminant comprenant
de la monensine ;
un alkylpolyglycoside ;
du monopropylèneglycol ;
de la gomme de xanthane ; et
de l'eau.

2. Formulation selon la revendication 1 comprenant en outre un ou plusieurs parmi
un agent ou système antimousse ;
un conservateur ;
un agent désamérisant ; ou
un système tamponnant le pH.

3. Formulation selon la revendication 1 dans laquelle ladite monensine est la monensine sodique.

4. Formulation selon la revendication 1 dans laquelle la quantité de monensine est de 3 % p/v à 50 % p/v.

5. Formulation selon la revendication 4 dans laquelle la quantité de monensine est de 3 % p/v à 20 % p/v.

6. Formulation selon la revendication 5 dans laquelle la quantité de monensine est d'environ 6 % p/v.

7. Formulation selon la revendication 1 comprenant
| | |
|---|---|
| Monensine sodique | 3 % - 50 % p/v, |
| Monopropylèneglcyol | 1 % - 20 % p/v, |
| Gomme de xanthane | 0,05 % - 5 % p/v, |
| Alkylpolyglycoside | 0,05 % - 2 % p/v, |
| Siméthicone | 0,05 % - 2 % p/v, |
| Dioxyde de silicium | 0,01 % - 1 % p/v, et |
| Eau jusqu'à 100 %. | |

8. Formulation selon la revendication 7 comprenant
| | |
|---|---|
| Monensine sodique | environ 6 % p/v, |
| Monopropylèneglcyol | environ 10 % p/v, |
| Phosphate disodique anhydre | environ 0,355 % p/v, |
| Phosphate monopotassique dihydraté | environ 0,04 % p/v, |
| Bromure de dialkyldiméthylammonium | environ 0,0064 % p/v, |
| Sorbitol | environ 3,5 % p/v, |
| Gomme de xanthane | environ 0,4 % p/v, |
| Alkylpolyglycoside | environ 0,5 % p/v, |
| Siméthicone | environ 0,333 % p/v, |
| Dioxyde de silicium | environ 0,167 % p/v, et |
| Eau jusqu'à 100 %. | |

9. Utilisation d'une formulation selon l'une quelconque des revendications 1 à 8 dans la fabrication d'un médicament conçu pour une administration orale à un animal ruminant, lequel médicament est pour
(A) chez le boeuf et les bovins laitiers,
le traitement ou la prévention de la cétose et/ou du météorisme,
l'amélioration de la production de lait, l'augmentation de la teneur en protéine de lait dans le lait, l'amélioration de l'absorption des minéraux, l'amélioration du gain de poids, et/ou l'amélioration de l'efficacité de la conversion d'aliments, le traitement ou la prévention de la coccidiose, et/ou
l'obtention d'avantages sur la reproduction, et/ou
(B) chez les veaux
l'une quelconque des applications de (A), et/ou
un lait de remplacement ou un additif au lait.

10. Procédé de formation d'une formulation selon l'une quelconque des revendications 1 à 8 qui comprend le mélange de la monensine avec un système aqueux contenant déjà au moins une certaine proportion de monopropylèneglycol, le conservateur éventuel (s'il est présent et s'il a un effet mouillant), et l'agent ou système antimousse, et
par la suite, le mélange dans le reste des composants (éventuellement avec un certain prémélange).
